# EUROPEAN PATENT SPECIFICATION

(11) **EP 1 505 816 B1**
(45) Date of publication and mention of the grant of the patent: **24.07.2013**
(21) Application number: 04254746.3
(22) Date of filing: 06.08.2004
(51) Int. Cl.: H04M 11/00, A61N 1/372

(54) **Data feedback loop for medical therapy adjustment**
Datenrückkoppelungsschleife für medizinische Therapieeinstellung
Boucle de rétroaction de données pour l'ajustement d'une thérapie médicale

(30) Priority: 08.08.2003 US 638065
(43) Date of publication of application: 09.02.2005
(73) Proprietor: PACESETTER, INC., Sylmar, CA 91342-9221 (US)
(72) Inventor: Badelt, Steven W., Granada Hills CA 91344 (US)
(74) Representative: Noble, Nicholas

(56) References cited:
- EP-A2- 1 062 984
- US-A- 3 769 965
- US-A- 5 544 661
- US-A- 5 720 770
- US-A1- 2002 143 372
- US-A1- 2003 013 945
- US-B1- 6 574 509
- DATABASE WPI Section EI, Week 200363 Derwent Publications Ltd., London, GB; Class T01, AN 2003-664444 XP002308196 & CN 1 428 696 A (YANG B) 9 July 2003 (2003-07-09)

## Description

This invention generally concerns implantable medical therapy devices and techniques related to gathering and processing data field-collected by such implantable medical therapy devices.

Implantable cardiac therapy devices (ICTDs) are implanted within the body of a patient to monitor, regulate, and/or correct heart function. ICTDs include implantable cardiac stimulation devices (e.g., implantable cardiac pacemakers, implantable defibrillators) that apply stimulation therapy to the heart as well as implantable cardiac monitors that monitor heart activity.

ICTDs typically include a control unit positioned within a casing that is implanted into the body and a set of leads that are positioned to impart stimulation and/or monitor cardiac activity. With improved processor and memory technologies, the control units have become increasingly more sophisticated, allowing them to monitor many types of conditions and apply tailored stimulation therapies in response to those conditions.

ICTDs are typically capable of being programmed remotely by an external programming device, often called a "programmer". Today, individual ICTDs are equipped with telemetry circuits that communicate with the programmer. One type of programmer utilizes an electromagnetic wand that is placed near the implanted cardiac device to communicate with the implanted device. When used in a sterile field, the wand may be enclosed in a sterile sheath. The wand contains a coil that forms a transformer coupling with the ICTD telemetry circuitry. The wand transmits low frequency signals by varying coil impedance.

Early telemetry systems were passive, meaning that the communication was unidirectional from the programmer to the implanted device. Passive telemetry allowed a treating physician to download instructions to the implanted device following implantation. Due to power and size constraints, early commercial versions of the implanted devices were incapable of transmitting information back to the programmer.

As power capabilities improved, active telemetry became feasible, allowing synchronous bi-directional communication between the implanted device and the programmer. Active telemetry utilizes a half-duplex communication mode in which the programmer sends instructions in a predefined frame format and, following termination of this transmission, the implanted device returns data using the frame format. With active telemetry, the treating physician is able to not only program the implanted device, but also retrieve information from the implanted device to evaluate heart activity and device performance. The treating physician may periodically want to review device performance or heart activity data for predefined periods of time to ensure that the device is providing therapy in desired manner. Consequently, current generation implantable cardiac therapy devices incorporate memories, and the processors periodically sample and record various performance parameter measurements in the memories.

Data pertaining to a patient's cardiac condition is gathered and stored by the programmer during programming sessions of the ICTDs. Analysis of the cardiac condition is performed locally by the programming software. Programmers offer comprehensive diagnostic capabilities, highspeed processing, and easy operation, thereby facilitating efficient programming and timely patient follow-up.

In addition to local analysis, TransTelephonic Monitoring (TTM) systems are employed to gather current cardiac data from patients who are remote from the healthcare provider. TTM systems are placed in patients' homes. They typically include a base unit that gathers information from the ICTD much like the programmer would. The base unit is connected to a telephone line so that data may be transmitted to the medical staff responsible for that patient. An example of an ICTD TTM system is a service from St. Jude Medical® and Raytel® Cardiac Services called "Housecall™." This service provides current programmed parameters and episode diagnostic information for a plurality of events including stored electrograms (EGMs). Real-time EGMs with annotated status information can also be transmitted.

Using a telephone and a transmitter, the TTM system provides both the medical staff and the patient the convenience of instant analysis of therapy without having the patient leave the comfort of home. Typically, real-time measurements are transmitted in just minutes. Patients may be closely monitored, and the medical staff has more control of their patient's treatment, thus administering better patient management.

One challenge that still persists, however, is how to efficiently and effectively present patient information and cardiac data to medical personnel and other knowledge workers who might have an interest in the device data. People utilize different types of computing devices to receive and view data, such as computers, portable computers, personal digital assistants, facsimile machines, and so on. These computing devices have different user interface capabilities and features. Accordingly, there is a need for a system that delivers the data to a wide variety of computing devices.

On an individual patient basis, there is no existing comprehensive and persistent mechanism for gathering long-term and real-time data. While ICTDs perform short-term monitoring and recording patient cardiac function, their relatively small processing and memory capabilities limit the thorough analysis, and action thereupon, of this data, particularly in the long term. Therefore, there is no existing mechanism for collecting data that includes long-term cardiac monitoring and treatment information of specific patients and other medical and demographic information of specific patients.

Although the TTM systems do monitor and transmit data for a specific patient, this data is not collected and stored in the ICTD itself in a manner that is persistent. Since there is limited memory capacity on the ICTD, the collected data is routinely wiped out of the ICTD's memory (typically, upon programming).

Generally, the ICTD does not analyze such data to automatically adjust patient therapy. While the ICTD may monitor some functions, such monitoring does not result in reprogramming the ICTD. Most conventional therapies monitor the patient and act upon the present circumstances, but do not change their function over the long term.

In addition, this data is not transmitted to a central database where it is analyzed to determine if a therapy adjustment is desirable; and if so, automatically indicating and reprogramming the ICTD to adjust the therapy accordingly.

On a basis of a patient population, there is no existing mechanism for gathering long-term and real-time data. There is no existing mechanism for collecting data that includes cardiac monitoring and treatment information of a patient population and other medical and demographic information of a patient population.

Although the TTM systems do monitor and transmit data for a specific patient, the data is not collected and stored regarding a patient population. With existing techniques and systems, patient population data - is not used to set "factory defaults" in the ICTD itself or the ICTD system itself. Conventionally, the ICTD does not use information derived from such patient population data to adjust patient therapy. Furthermore, with traditional techniques, patient population data is not analyzed to determine if therapy adjustment is desirable for a specific patient.

A system according to the preamble of claim 1 is known from US 5 720 770.

One challenge that still persists, however, is how to collect and analyze data―which includes historical cardiac-status-and-treatment information―for a specific patient and to adjust a patient's therapy accordingly. Likewise, there is a challenge to collect and analyze data― which includes historical cardiac-status-and-treatment information―for a patient population and to adjust a patient's therapy accordingly.

According to the invention, there is provided apparatus for administering cardiac therapy to a plurality of patients comprising: a plurality of implantable cardiac therapy devices configured to be implanted in corresponding patients, a central database and a communications link configured to facilitate communications between the central database and the plurality of implantable cardiac therapy devices, and whereby the database is operative to receive data relating to therapy administered to the patients by the plurality of implantable cardiac therapy devices, process the collection of data to discover patterns, relationships, or correlations between such data, and to modify therapy of one or more of the implantable cardiac therapy devices in response to the patterns, relationships, or correlations between the data.

Preferably, the data comprises historical cardiac-status-and-treatment information, or historical cardiac-status-and-treatment information of a plurality of patients.

Preferably, the means carrying out the processing comprises a knowledge discovery in database (KDD) system.

Preferably, the data comprises one or more parameters selected from a group of parameters consisting of:
- cardiac monitoring data and statistics;
- cardiac treatment data and statistics;
- ICTD model and serial number;
- ICTD battery status;
- waveform of treatment;
- shocking protocol;
- patient age;
- patient gender;
- patient activity patterns;
- patient weight;
- patient ethnic/nationality;
- patient genetics;
- patient temporal cycles;
- patient sleep/wake patterns;
- patient medications.

Described herein is a technology for implantable medical therapy devices and techniques related to gathering and processing data field-collected by such implantable medical therapy devices.

With an ICTD system and a central database, at least one embodiment of the invention, described herein, collects and analyzes data―which includes historical cardiac-status-and-treatment information― for a specific patient and adjusts a patient's therapy accordingly. With an ICTD system and a central database, at least one embodiment of the invention, described herein, collects and analyzes data―which includes historical cardiac-status-and-treatment information―for a patient population and adjusts a patient's therapy accordingly.

The invention enables a method comprising: administering cardiac therapy to a plurality of patients via a plurality of implanted cardiac therapy devices; storing data regarding the therapy at the respective implanted cardiac therapy devices; collecting the data from the plurality of implanted cardiac therapy devices; processing the collected data to discover patterns, relationships, or correlations between such data; modifying therapy of one or more patients based upon the patterns, relationships, or correlations.

The invention also permits a method comprising: administering cardiac therapy to a plurality of patients; gathering data from each patient regarding the therapy and the response to the therapy; collecting data related to such therapy and the response from the plurality of patients; analyzing the data to determine appropriate therapies; and modifying therapy of one or more patients based upon the patterns, relationships, or correlations. The therapies can then be adjusted for one or more patients in response to the analyzing. The analyzing may comprise statistically processing the collection of data to discover patterns, relationships, or correlations between such data. The collecting may comprise field-collecting the collection of data from a plurality of implantable cardiac devices (ICTDs).

Further features and advantages of the claimed invention can be more readily understood by reference to the following description of specific embodiments taken in conjunction with the accompanying drawings, in which:
Fig. 1 is a diagrammatic illustration of a cardiac therapy network architecture with an implantable cardiac therapy device (ICTD) connected to a network of computing systems used by various knowledge workers;
Fig. 2 is a functional diagram illustrating information flow from the ICTD to the computing systems associated with the knowledge workers;
Fig. 3 is a functional diagram illustrating how the various computing systems share pieces of information to improve care given to the patient;
Fig. 4 is a functional diagram illustrating a patient feedback architecture and the information flow from the computing systems back to the ICTD;
Fig. 5 is a simplified illustration of an ICTD in electrical communication with a patient's heart for monitoring heart activity and/or delivering stimulation therapy;
Fig. 6 is a functional block diagram of an exemplary implantable cardiac therapy device;
Fig. 7 is a functional block diagram of an exemplary computing device that may be used in the computing systems of the cardiac therapy network architecture; and
Fig. 8 is a flow diagram that describes a methodological implementation in accordance with one embodiment.

### Detailed description

In the following description, for purposes of the explanation, specific numbers, materials and configurations are set forth in order to provide a thorough understanding of the present invention. However, it will be apparent to one skilled in the art that the present invention may be practiced without the specific exemplary details. In other instances, well-known features are omitted or simplified to clarify the description of the exemplary implementations of present invention. Furthermore, for ease of understanding, certain method steps are delineated as separate steps; however, these separately delineated steps should not be construed as necessarily order dependent in their performance.

The following description sets forth one or more exemplary implementations of a Data Feedback Loop for Medical Therapy Adjustment that incorporate elements recited in the appended claims. These implementations are described with specificity in order to meet statutory written description, enablement, and best-mode requirements. However, the description itself is not intended to limit the scope of this patent.

### Overview

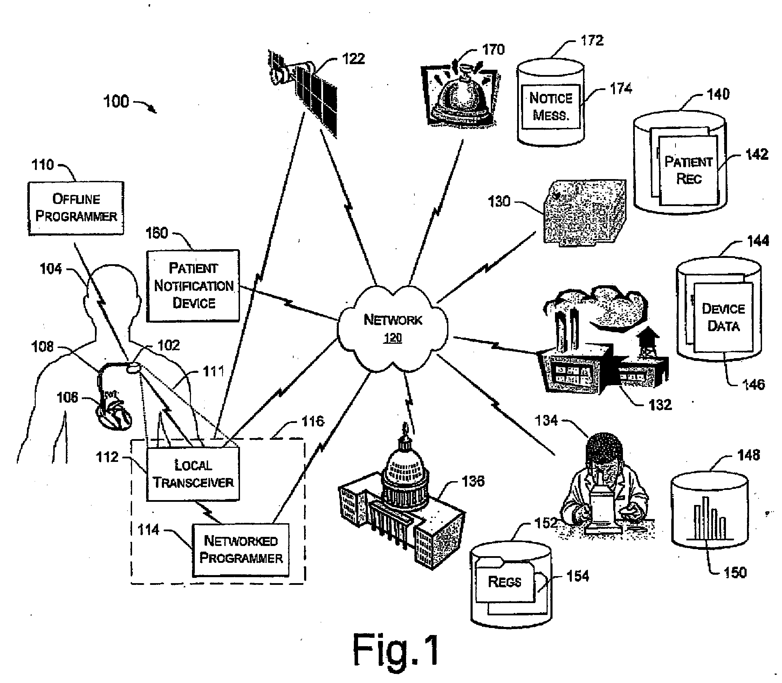

The one or more exemplary implementations, described herein, of the present invention may be implemented (in whole or in part) by an exemplary cardiac therapy network architecture 100 like that shown in Fig. 1.

With at least one embodiment, the exemplary feedback loop technology collects and analyzes data―which includes historical cardiac-status-and-treatment information―for a specific patient and adjusts a patient's therapy accordingly. Rather than retaining history of a patient from a programming session or a collection of such sessions, the patient-specific data is collected over the lifetime of the patient―as long as the patient has an ICTD implanted therein. This data is "field-collected." At least in part, this means that the data is collected outside of a laboratory or office-type setting. Field-collected data is obtained while the patient goes about their normal activities.

With at least one embodiment, the exemplary feedback loop technology collects and analyzes data―which includes historical cardiac-status-and-treatment information―for a patient population and adjusts a patient's therapy accordingly. Rather than retaining history of a single patient from a single programming session or a collection of such sessions for a single patient, the patient-population data is collected over the lifetime of a population of patients. This data is also "field-collected."

In other words, the technology described herein provides a personalized closed-feedback loop for individual patients and/or a generalized closed-feedback loop for patient populations. With the personalized loop, a patient's treatment is automatically adjusted, or recommended to be adjusted, based upon the patient's own medical history (including cardiac monitoring and response to cardiac treatment) and other personal data. With the generalized loop, a patient's treatment is automatically adjusted based upon the medical history (including cardiac monitoring and response to cardiac treatment) and other personal data of a patient population―particularly where correlations exist between the subject patient and those in the population.

### Cardiac Therapy Network

Fig. 1 shows an exemplary cardiac therapy network architecture 100 that includes an implantable cardiac therapy device (ICTD) 102 coupled to a network of computing systems associated with various knowledge workers who have interest in cardiac therapy. The ICTD is illustrated as being implanted in a human patient 104. The ICTD 102 is in electrical communication with a patient's heart 106 by way of multiple leads 108 suitable for monitoring cardiac activity and/or delivering multi-chamber stimulation and shock therapy.

The ICTD 102 may communicate with a standalone or offline programmer 110 via short-range telemetry technology. The offline programmer 110 is equipped with a wand that, when positioned proximal to the ICTD 102, communicates with the ICTD 102 through a magnetic coupling.

The ICTD 102 can alternatively, or additionally, communicate with a local transceiver 112. The local transceiver 112 may be a device that resides on or near the patient, such as an electronic communications device that is worn by the patient or is situated on a structure within the room or residence of the patient. The local transceiver 112 communicates with the ICTD 102 using short-range telemetry or longer-range high-frequency-based telemetry, such as RF (radio frequency) transmissions. Alternatively, the local transceiver 112 may be incorporated into the ICTD 102, as represented by dashed line 111. In this case, the ICTD includes a separate and isolated package area that accommodates high-frequency transmissions without disrupting operation of the monitoring and stimulation circuitry.

Depending upon the implementation and transmission range, the local transceiver 112 can be in communication with various other devices of the network architecture 100. One possible implementation is for the local transceiver 112 to transmit information received from the ICTD 102 to a networked programmer 114, which is connected to network 120. The networked programmer 114 is similar in operation to standalone programmer 110, but differs in that it is connected to the network 120. The networked programmer 114 may be local to, or remote from, the local transceiver 112; or alternatively, the local transceiver 112 may be incorporated into the networked programmer 114, as represented by dashed line 116.

Another possible implementation is for the local transceiver to be connected directly to the network 120 for communication with remote computing devices and/or programmers. Still another possibility is for the local transceiver 112 to communicate with the network 120 via wireless communication, such as via a satellite system 122.

The network 120 may be implemented by one or more different types of networks (e.g., Internet, local area network, wide area network, telephone, cable, satellite, etc.), including wire-based technologies (e.g., telephone line, cable, fiber optics, etc.) and/or wireless technologies (e.g., RF, cellular, microwave, IR, wireless personal area network, etc.). The network 120 can be configured to support any number of different protocols, including HTTP (HyperText Transport Protocol), TCP/IP (Transmission Control Protocol/Internet Protocol), WAP (Wireless Application Protocol), Bluetooth, and so on.

A number of knowledge workers are interested in data gathered from the implantable cardiac therapy device 102. Representative knowledge workers include healthcare providers 130, the device manufacturer 132, clinical groups 134, and regulatory agencies 136. The knowledge workers are interested in different portions of the data. For instance, the healthcare providers 130 are interested in information pertaining to a particular patient's condition. The manufacturer 132 cares about how the device is operating. The clinical groups 134 want certain data for inclusion in patient populations that can be studied and analyzed. The regulatory agencies 136 are concerned whether the devices, and various treatments administered by them, are safe or pose a health risk.

The network architecture 100 facilitates distribution of the device data to the various knowledge workers. Information gathered from the device is integrated, processed, and distributed to the knowledge workers. Computer systems maintain and store the device data, and prepare the data for efficient presentation to the knowledge workers. The computer systems are represented pictorially in Fig. 1 as databases. However, such system can be implemented using a wide variety of computing devices, ranging from small handheld computers or portable digital assistants (PDAs) carried by physicians to workstations or mainframe computers with large storage capabilities. The healthcare providers 130 are equipped with computer systems 140 that store and process patient records 142. The manufacturer 132 has a computer system 144 that tracks device data 146 returned from ICTDs 102. The clinical groups 134 have computer systems 148 that store and analyze data across patient populations, as represented by a histogram 150. The regulatory agencies 136 maintain computer systems 152 that register and track healthcare risk data 154 for ICTDs.

The network architecture 100 supports two-way communication. Not only is data collected from the ICTD 102 and distributed to the various computer systems of the knowledge workers, but also information can be returned from these computer systems to the networked programmer 114 and/or the local transceiver 112 for communication back to the ICTD 102. Information returned to the ICTD 102 may be used to adjust operation of the device, or modify therapies being applied by the device. Such information may be imparted to the ICTD 102 automatically, without the patient's knowledge.

Additionally, information may be sent to a patient notification device 160 to notify the patient of some event or item. The patient notification device 160 can be implemented in a number of ways including, for example, as a telephone, a cellular phone, a pager, a PDA (personal digital assistant), a dedicated patient communication device, a computer, an alarm, and so on. Notifications may be as simple as an instruction to sound an alarm to inform the patient to call into the healthcare providers, or as complex as HTML-based pages with graphics and textual data to educate the patient. Notification messages sent to the patient notification device 160 can contain essentially any type of information related to cardiac medicinal purposes or device operation. Such information might include new studies released by clinical groups pertaining to device operation and patient activity (e.g., habits, diets, exercise, etc.), recall notices or operational data from the manufacturer, patient-specific instructions sent by the healthcare providers, or warnings published by regulatory groups.

Notifications can be sent directly from the knowledge worker to the patient. Additionally, the network architecture 100 may include a notification system 170 that operates computer systems 172 designed to create and deliver notification messages 174 on behalf of the knowledge workers. The notification system 170 delivers the messages in formats supported by the various types of patient notification devices 160. For instance, if the patient carries a pager, a notification message might consist of a simple text statement in a pager protocol. For a more sophisticated wireless-enabled PDA or Internet-oriented cellular phone, messages might contain more than text data and be formatted using WAP formats.

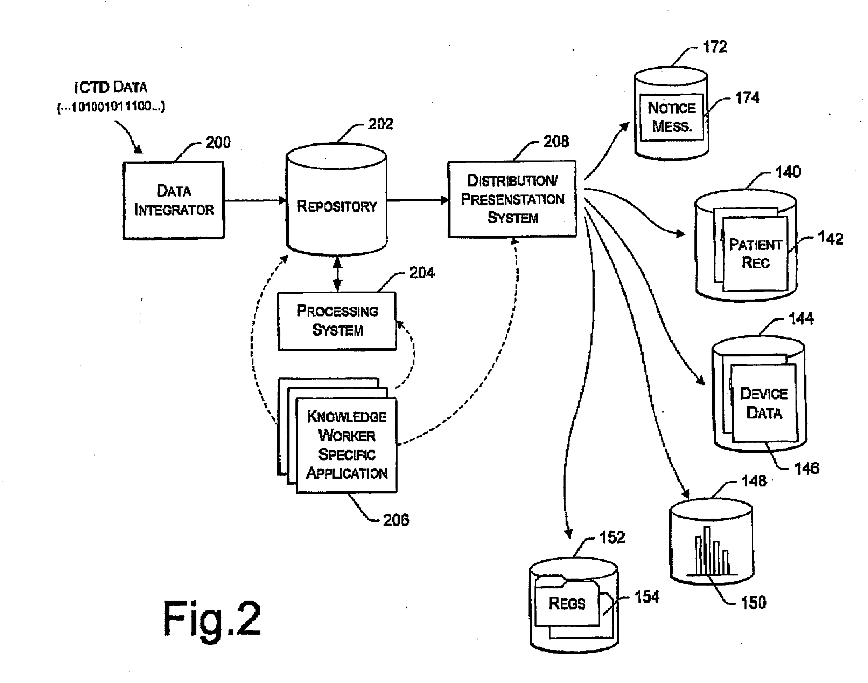

Fig. 2 shows the flow of data from the implantable cardiac therapy device 102 to the various computer systems used by the knowledge workers. Data from the ICTD is output as digital data, as represented by the string of 0's and 1's. The data may consist of any number of items, including heart activity (e.g., ECG), patient information, device operation, analysis results from on-device diagnostics, and so on.

A data integrator 200 accumulates the data and stores it in a repository 202. A processing system 204 processes portions of the data according to various applications 206 that are specifically tailored to place the data into condition for various knowledge workers. For example, healthcare workers might be interested in certain portions of the data, such as the ECG data and the patient information. Clinical scientists might be interested in the heart data, but do not wish to see any patient information. Manufacturers may be interested in the raw data stream itself as a tool to discern how the device is operating. Depending on the needs of the end worker, the processing system 204 takes the raw device data, evaluates its accuracy and completeness, and generates different packages of data for delivery to the various knowledge workers. The processed data packages are also stored in the repository 202.

When the data is ready for delivery, a distribution/presentation system 208 distributes the different packages to the appropriate computer systems 140, 144, 148, 152, and 172. The distribution/presentation system 208 is configured to serve the packages according to the protocols and formats desired by the computer systems. In this manner, the network architecture 100 allows relevant portions of device data, collected from the ICTD, to be disseminated to the appropriate knowledge workers in a form they prefer.

Once the ICTD data is delivered, the computer systems 140, 144, 148, 152, and 172 store the data and/or present the data to the knowledge worker. The computer systems may perform further processing specific to their use of the data. Through these processes, the knowledge workers create additional information that is useful to the patient, or other knowledge workers with interests in ICTDs. For example, from the ICTD data, the knowledge workers might devise improved therapies for a given patient, or create instructions to modify operation of a specific ICTD, or gain a better understanding of how implantable cardiac devices operate in general, or develop better technologies for future generations of ICTDs. Much of this created knowledge can be shared among the various knowledge workers.

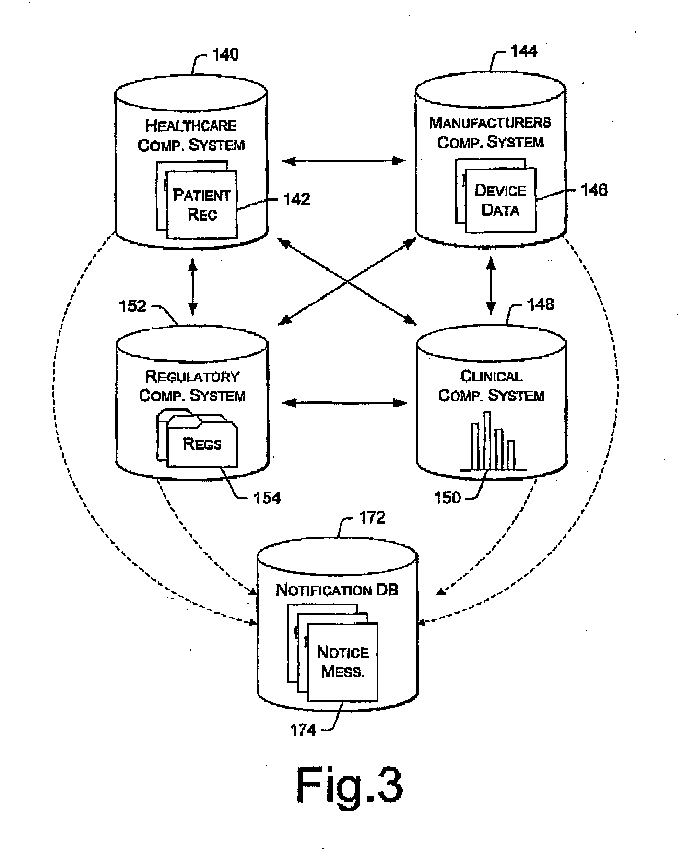

Fig. 3 shows how the various computing systems 140, 144, 148, 152, and 172 can cooperate and share pieces of information to improve the care given to a patient. Where appropriate and legally acceptable, the computer systems may be configured to pass non-private information among the various knowledge workers to better improve their understanding of the implantable medical field. Clinical results 150 produced by the clinical computer systems 148 may be shared with healthcare providers to improve patient care or with manufacturers to help in their design of next generation devices. The sharing of information may further lead to better and timelier healthcare for the patients.

If the collective knowledge base produces information that may prove helpful to the patient, that information can be passed to the notification system 172 for delivery to one or more patients. Also, any one of the knowledge workers may wish to employ the notification system 172 to send messages to the patient(s).

### Patient Feedback Architecture

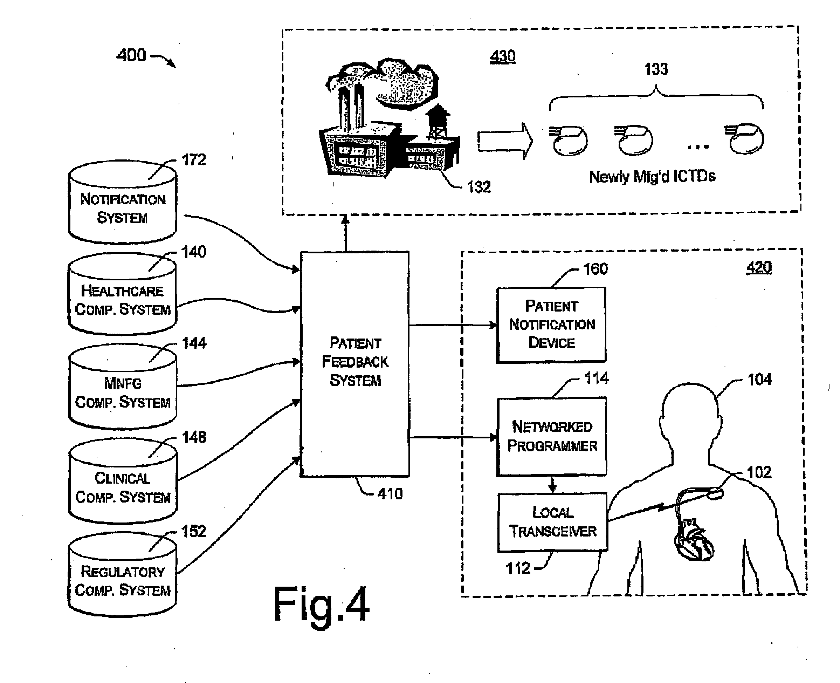

Fig. 4 shows the patient feedback architecture 400. It also shows, in more detail, the flow of information back from the various computer systems used by the knowledge workers to the implantable cardiac therapy device 102; the patient notification device 160 or back to the device manufacturer 132.

Information from any one of the computing systems―healthcare computer system(s) 140, manufacturer computer system(s) 144, clinical computer system(s) 148, regulatory computer system(s) 152―or the notification system 172 can be sent to a patient feedback system 410.

As shown by arrows between the patient feedback system 410 and dashed box 420, the system 410 facilitates delivery of the information back to the patient. As shown by arrows between the patient feedback system 410 and dashed box 430, the system 410 facilitates delivery of the information back to the device manufacturer 132.

The patient feedback system may be an independent system, or incorporated into one or more of the computing systems. It may alternatively be integrated into the notification system 172.

The patient feedback system 410 may be implemented in many ways. As one exemplary implementation, the patient feedback system 410 is implemented as a server that serves content back to the networked programmer 114, which then uses the information to program the ICTD 102 through a built in transceiver 116, local transceiver 112, or wand-based telemetry. As another possible implementation, the patient feedback system may be a cellular or RF transmission system that sends information back to the patient feedback device 160.

The network architecture 100 facilitates continuous care around the clock, regardless of where the patient is located. For instance, suppose the patient is driving in the car when a cardiac episode occurs. The ICTD 102 detects the condition and transmits an alert message about the condition to the local transceiver 112. The message is processed and delivered to a physician's computer or PDA via the network 120. The physician can make a diagnosis and send some instructions back to the patient's ICTD. The physician might also have a notification message that guides the patient to a nearest healthcare facility for further treatment sent via the notification system 170 to the patient's notification device 160. Concurrently, the physician can share the patient's records online with an attending physician at the healthcare facility so that the attending physician can review the records prior to the patient's arrival.

With the patient feedback architecture 400, the patient feedback system 410 collects data for a specific patient. The patient feedback system 410 may process such data. The system may transmit such data to another component, such as the networked programmer 114, for processing or additional processing of the data.

The patient feedback system 410 (or another component) may determine a change in therapy for the specific patient based upon data gathered from that patient. The determination may be noted and documented. It may be transmitted to the patient notification device 160. It may be transmitted to the networked programmer 114.

Furthermore, the change of therapy may be made automatically by communicating to the ICTD 102 via the local transceiver 112 or other such mechanisms.

Alternatively, with the patient feedback architecture 400, the patient feedback system 410 collects data for a patient population (i.e., more than one patient). The patient feedback system 410 may process such data. The system may transmit such data to another component, such as the networked programmer 114, for processing or additional processing of the data.

The patient feedback system 410 (or another component) may determine a change in therapy for the specific patient based upon data from a patient population. The determination may be noted and documented. It may be transmitted to the patient notification device 160. It may be transmitted to the networked programmer 114.

Furthermore, the change of therapy may be made automatically by communicating to the ICTD 102 via the local transceiver 112 or other such mechanisms.

More alternatively still, with the patient feedback architecture 400, the patient feedback system 410 collects data for a patient population (i.e., more than one patient). The patient feedback system 410 may process such data. The system may transmit such data to another component, such as a part of the device manufacturer 132, for processing or additional processing of the data.

Based upon the data, the patient feedback system 410 (or another component) may determine to alter manufacturer's default settings (i.e., configuration) of newly manufactured ICTDs, such as ICTDs 133 of Fig. 4. In other words, defaults, constants, and formulas employed by ICTDs may be modified based upon processed population data alone or based upon processed population data and information regarding a specific patient of a particular ICTD.

### Exemplary ICTD

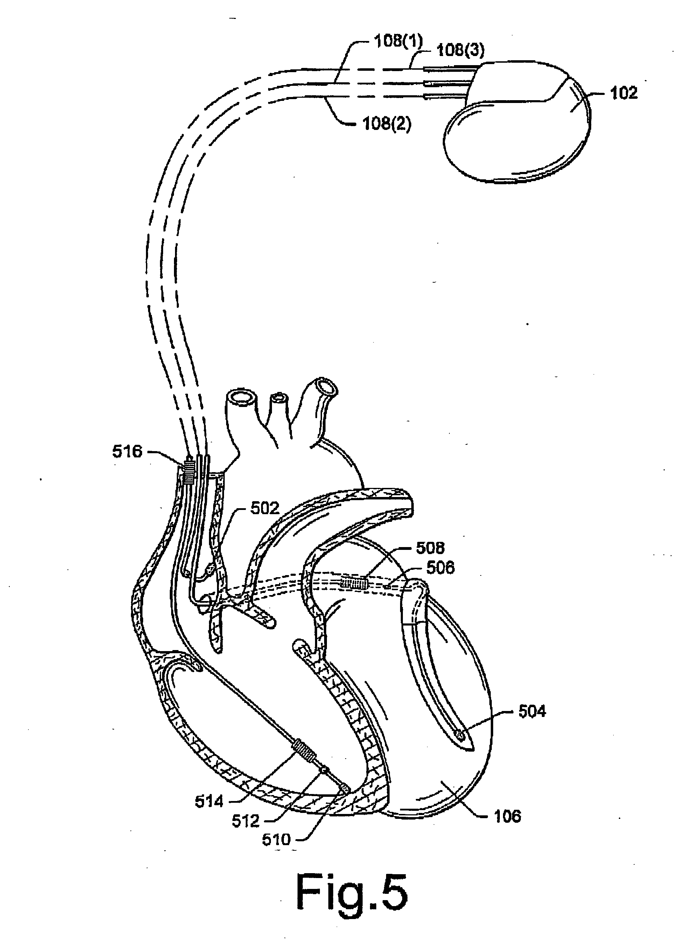

Fig. 5 shows an exemplary ICTD 102 in electrical communication with a patient's heart 106 for monitoring heart activity and/or delivering stimulation therapy, such as pacing or defibrillation therapies. The ICTD 102 is in electrical communication with a patient's heart 106 by way of three leads 108(1)-(3). To sense atrial cardiac signals and to provide right atrial chamber stimulation therapy, the ICTD 102 is coupled to an implantable right atrial lead 108(1) having at least an atrial tip electrode 502, which typically is implanted in the patient's right atrial appendage. To sense left atrial and ventricular cardiac signals and to provide left chamber pacing therapy, the ICTD 102 is coupled to a coronary sinus lead 108(2) designed for placement in the coronary sinus region via the coronary sinus. The coronary sinus lead 108(2) positions a distal electrode adjacent to the left ventricle and/or additional electrode(s) adjacent to the left atrium. An exemplary coronary sinus lead 108(2) is designed to receive atrial and ventricular cardiac signals and to deliver left ventricular pacing therapy using at least a left ventricular tip electrode 504, left atrial pacing therapy using at least a left atrial ring electrode 506, and shocking therapy using at least a left atrial coil electrode 508.

The ICTD 102 is also shown in electrical communication with the patient's heart 106 by way of an implantable right ventricular lead 108(3) having, in this implementation, a right ventricular tip electrode 510, a right ventricular ring electrode 512, a right ventricular (RV) coil electrode 514, and an SVC coil electrode 516. Typically, the right ventricular lead 108(3) is transvenously inserted into the heart 102 to place the right ventricular tip electrode 510 in the right ventricular apex so that the RV coil electrode 514 will be positioned in the right ventricle and the SVC coil electrode 516 will be positioned in the superior vena cava. Accordingly, the right ventricular lead 108(3) is capable of receiving cardiac signals, and delivering stimulation in the form of pacing and shock therapy to the right ventricle.

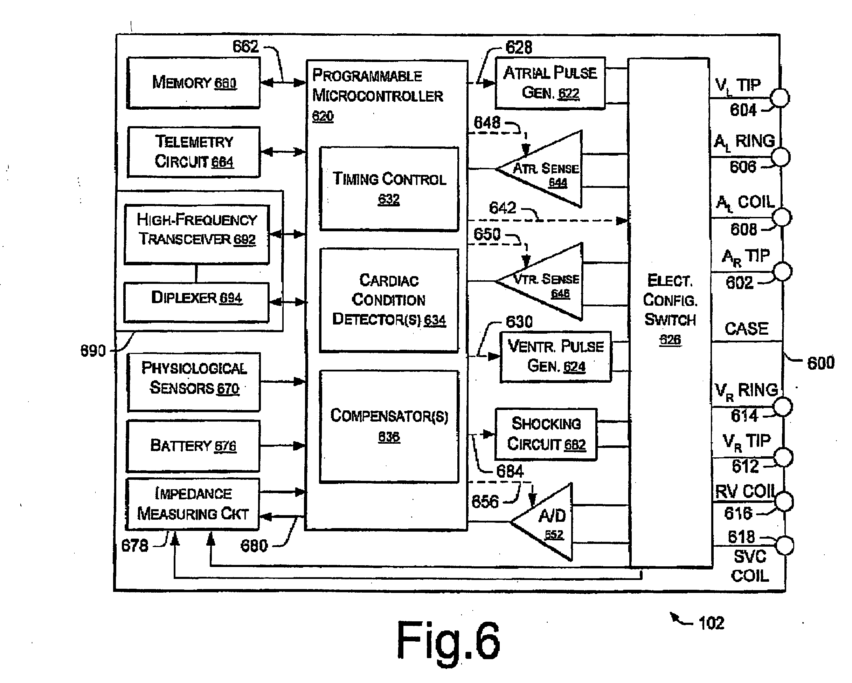

Fig. 6 shows an exemplary, simplified block diagram depicting various components of the ICTD 102. The ICTD 102 can be configured to perform one or more of a variety of functions including, for example, monitoring heart activity, monitoring patient activity, and treating fast and slow arrhythmias with stimulation therapy that includes cardioversion, defibrillation, and pacing stimulation. While a particular multi-chamber device is shown, it is to be appreciated and understood that this is done for illustration purposes.

The circuitry is housed in housing 600, which is often referred to as the "can", "case", "encasing", or "case electrode", and may be programmably selected to act as the return electrode for unipolar modes. Housing 600 may further be used as a return electrode alone or in combination with one or more of the coil electrodes for shocking purposes. Housing 600 further includes a connector (not shown) having a plurality of terminals 602, 604, 606, 608, 612, 614, 616, and 618 (shown schematically and, for convenience, the names of the electrodes to which they are connected are shown next to the terminals).

To achieve right atrial sensing and pacing, the connector includes at least a right atrial tip terminal (A_{R} TIP) 602 adapted for connection to the atrial tip electrode 502. To achieve left chamber sensing, pacing, and shocking, the connector includes at least a left ventricular tip terminal (V_{L} TIP) 604, a left atrial ring terminal (A_{L} RING) 606, and a left atrial shocking terminal (A_{L} COIL) 608, which are adapted for connection to the left ventricular ring electrode 504, the left atrial ring electrode 506, and the left atrial coil electrode 508, respectively. To support right chamber sensing, pacing, and shocking, the connector includes a right ventricular tip terminal (V_{R} TIP) 612, a right ventricular ring terminal (V_{R} RING) 614, a right ventricular shocking terminal (RV COIL) 616, and an SVC shocking terminal (SVC COIL) 618, which are adapted for connection to the right ventricular tip electrode 510, right ventricular ring electrode 512, the RV coil electrode 514, and the SVC coil electrode 516, respectively.

At the core of the ICTD 102 is a programmable microcontroller 620 that controls various operations of the ICTD, including cardiac monitoring and stimulation therapy. Microcontroller 620 includes a microprocessor (or equivalent control circuitry), RAM and/or ROM memory, logic and timing circuitry, state machine circuitry, and I/O circuitry. Microcontroller 620 includes the ability to process or monitor input signals (data) as controlled by a program code stored in a designated block of memory. Any suitable microcontroller 620 may be used. The use of microprocessor-based control circuits for performing timing and data analysis functions are well known in the art.

For discussion purposes, microcontroller 620 is illustrated as including timing control circuitry 632 to control the timing of the stimulation pulses (e.g., pacing rate, atrio-ventricular (AV) delay, atrial interconduction (A-A) delay, or ventricular interconduction (V-V) delay, etc.) as well as to keep track of the timing of refractory periods, blanking intervals, noise detection windows, evoked response windows, alert intervals, marker channel timing, and so on. Microcontroller 220 may further include various types of cardiac condition detectors 634 (e.g., an arrhythmia detector, a morphology detector, etc.) and various types of compensators 636 (e.g., orthostatic compensator, syncope response module, etc.). These components can be utilized by the device 102 for determining desirable times to administer various therapies. The components 632-636 may be implemented in hardware as part of the microcontroller 620, or as software/firmware instructions programmed into the device and executed on the microcontroller 620 during certain modes of operation.

The ICTD 102 further includes an atrial pulse generator 622 and a ventricular pulse generator 624 that generate pacing stimulation pulses for delivery by the right atrial lead 108(1), the coronary sinus lead 108(2), and/or the right ventricular lead 108(3) via an electrode configuration switch 626. It is understood that in order to provide stimulation therapy in each of the four chambers of the heart, the atrial and ventricular pulse generators, 622 and 624, may include dedicated, independent pulse generators, multiplexed pulse generators, or shared pulse generators. The pulse generators 622 and 624 are controlled by the microcontroller 620 via appropriate control signals 628 and 630, respectively, to trigger or inhibit the stimulation pulses.

The electronic configuration switch 626 includes a plurality of switches for connecting the desired electrodes to the appropriate I/O circuits, thereby providing complete electrode programmability. Accordingly, switch 626, in response to a control signal 642 from the microcontroller 620, determines the polarity of the stimulation pulses (e.g., unipolar, bipolar, combipolar, etc.) by selectively closing the appropriate combination of switches (not shown).

Atrial sensing circuits 644 and ventricular sensing circuits 646 may also be selectively coupled to the right atrial lead 108(1), coronary sinus lead 108(2), and the right ventricular lead 108(3), through the switch 626 to detect the presence of cardiac activity in each of the four chambers of the heart. Accordingly, the atrial (ATR. SENSE) and ventricular (VTR. SENSE) sensing circuits, 644 and 646, may include dedicated sense amplifiers, multiplexed amplifiers, or shared amplifiers. Each sensing circuit 644 and 646 may further employ one or more low power, precision amplifiers with programmable gain and/or automatic gain control, bandpass filtering, and a threshold detection circuit to selectively sense the cardiac signal of interest. The automatic gain control enables the ICTD 102 to deal effectively with the difficult problem of sensing the low amplitude signal characteristics of atrial or ventricular fibrillation. Switch 626 determines the "sensing polarity" of the cardiac signal by selectively closing the appropriate switches. In this way, the clinician may program the sensing polarity independent of the stimulation polarity.

The outputs of the atrial and ventricular sensing circuits 644 and 646 are connected to the microcontroller 620 which, in turn, is able to trigger or inhibit the atrial and ventricular pulse generators 622 and 624, respectively, in a demand fashion in response to the absence or presence of cardiac activity in the appropriate chambers of the heart. The sensing circuits 644 and 646 receive control signals over signal lines 648 and 650 from the microcontroller 620 for purposes of controlling the gain, threshold, polarization charge removal circuitry (not shown), and the timing of any blocking circuitry (not shown) coupled to the inputs of the sensing circuits 644 and 646.

Cardiac signals are also applied to inputs of an analog-to-digital (A/D) data acquisition system 652. The data acquisition system 652 is configured to acquire intracardiac electrogram signals, convert the raw analog data into a digital signal, and store the digital signals for later processing and/or telemetric transmission to an external device 654. The data acquisition system 652 is coupled to the right atrial lead 108(1), the coronary sinus lead 108(2), and the right ventricular lead 108(3) through the switch 626 to sample cardiac signals across any pair of desired electrodes.

The data acquisition system 652 may be coupled to the microcontroller 620, or other detection circuitry, to detect an evoked response from the heart 106 in response to an applied stimulus, thereby aiding in the detection of "capture". Capture occurs when an electrical stimulus applied to the heart is of sufficient energy to depolarize the cardiac tissue, thereby causing the heart muscle to contract. The microcontroller 620 detects a depolarization signal during a window following a stimulation pulse, the presence of which indicates that capture has occurred. The microcontroller 620 enables capture detection by triggering the ventricular pulse generator 624 to generate a stimulation pulse, starting a capture detection window using the timing control circuitry 632 within the microcontroller 620, and enabling the data acquisition system 652 via control signal 656 to sample the cardiac signal that falls in the capture detection window and, based on the amplitude, determines if capture has occurred.

The microcontroller 620 is further coupled to a memory 660 by a suitable data/address bus 662, wherein the programmable operating parameters used by the microcontroller 620 are stored and modified, as required, in order to customize the operation of the implantable device 102 to suit the needs of a particular patient. Such operating parameters define, for example; pacing pulse amplitude, pulse duration, electrode polarity, rate, sensitivity, automatic features, arrhythmia detection criteria, and the amplitude, waveshape and vector of each shocking pulse to be delivered to the patient's heart 106 within each respective tier of therapy. With memory 660, the ICTD 102 is able to sense and store a relatively large amount of data (e.g., from the data acquisition system 652), which may transmitted to the external network of knowledge workers for subsequent analysis.

Operating parameters of the ICTD 102 may be non-invasively programmed into the memory 660 through a telemetry circuit 664 in telemetric communication with an external device, such as a programmer 110 or local transceiver 112. The telemetry circuit 664 advantageously allows intracardiac electrograms and status information relating to the operation of the device 102 (as contained in the microcontroller 620 or memory 660) to be sent to the external devices.

The ICTD 100 can further include one or more physiologic sensors 670, commonly referred to as "rate-responsive" sensors because they are typically used to adjust pacing stimulation rate according to the exercise state of the patient. However, the physiological sensor 670 may further be used to detect changes in cardiac output, changes in the physiological condition of the heart, or diurnal changes in activity (e.g., detecting sleep and wake states, detecting position or postural changes, etc.). Accordingly, the microcontroller 620 responds by adjusting the various pacing parameters (such as rate, AV Delay, V-V Delay, etc.) at which the atrial and ventricular pulse generators, 622 and 624, generate stimulation pulses. While shown as being included within the device 102, it is to be understood that the physiologic sensor 670 may also be external to the device 102, yet still be implanted within or carried by the patient. Examples of physiologic sensors that may be implemented in device 102 include known sensors that, for example, sense respiration rate and/or minute ventilation, pH of blood, ventricular gradient, and so forth.

The ICTD 102 additionally includes a battery 676 that provides operating power to all of circuits shown in Fig. 2. If the device 102 is configured to deliver pacing or shocking therapy, the battery 676 is capable of operating at low current drains for long periods of time (e.g., preferably less than 10 µA), and is capable of providing high-current pulses (for capacitor charging) when the patient requires a shock pulse (e.g., preferably, in excess of 2 A, at voltages above 2 V, for periods of 10 seconds or more). The battery 676 also desirably has a predictable discharge characteristic so that elective replacement time can be detected. As one example, the device 102 employs lithium/silver vanadium oxide batteries.

The ICTD 102 can further include magnet detection circuitry (not shown), coupled to the microcontroller 620, to detect when a magnet is placed over the device 102. A magnet may be used by a clinician to perform various test functions of the device 102 and/or to signal the microcontroller 620 that the external programmer is in place to receive or transmit data to the microcontroller 620 through the telemetry circuits 664.

The ICTD 102 further includes an impedance measuring circuit 678 that is enabled by the microcontroller 620 via a control signal 680. Uses for an impedance measuring circuit 678 include, but are not limited to, lead impedance surveillance during the acute and chronic phases for proper lead positioning or dislodgement; detecting operable electrodes and automatically switching to an operable pair if dislodgement occurs; measuring respiration or minute ventilation; measuring thoracic impedance for determining shock thresholds; detecting when the device has been implanted; measuring stroke volume; and detecting the opening of heart valves, etc. The impedance measuring circuit 678 is advantageously coupled to the switch 626 so that any desired electrode may be used.

In the case where the device 102 is intended to operate as an implantable cardioverter/defibrillator (ICD) device, it detects the occurrence of an arrhythmia, and automatically applies an appropriate electrical shock therapy to the heart aimed at terminating the detected arrhythmia. To this end, the microcontroller 620 further controls a shocking circuit 682 by way of a control signal 684. The shocking circuit 682 generates shocking pulses of low (up to 0.5 Joules), moderate (0.5 - 10 Joules), or high energy (11 to 40 Joules), as controlled by the microcontroller 620. Such shocking pulses are applied to the patient's heart 106 through at least two shocking electrodes, and as shown in this implementation, selected from the left atrial coil electrode 508, the RV coil electrode 514, and/or the SVC coil electrode 516. As noted above, the housing 600 may act as an active electrode in combination with the RV coil electrode 514, or as part of a split electrical vector using the SVC coil electrode 516 or the left atrial coil electrode 508 (i.e., using the RV electrode as a common electrode).

Cardioversion shocks are generally considered to be of low to moderate energy level (so as to minimize pain felt by the patient), and/or synchronized with an R-wave and/or pertaining to the treatment of tachycardia. Defibrillation shocks are generally of moderate to high energy level (i.e., corresponding to thresholds in the range of 5-40 Joules), delivered asynchronously (since R-waves may be too disorganized), and pertaining exclusively to the treatment of fibrillation. Accordingly, the microcontroller 620 is capable of controlling the synchronous or asynchronous delivery of the shocking pulses.

The ICTD 102 may further be designed with the ability to support high-frequency wireless communication, typically in the radio frequency (RF) range. As illustrated in Fig. 2, the can 600 may be configured with a secondary, isolated casing 690 that contains circuitry for handling high-frequency signals. Within this separate case 690 are a high-frequency transceiver 692 and a diplexer 694. High-frequency signals received by a dedicated antenna, or via leads 108, are passed to the transceiver 692. Due to the separate casing region 690, the transceiver handles the high-frequency signals in isolation apart from the cardiac therapy circuitry. In this manner, the high-frequency signals can be safely handled, thereby improving telemetry communication, without adversely disrupting operation of the other device circuitry.

### Exemplary Computing Device

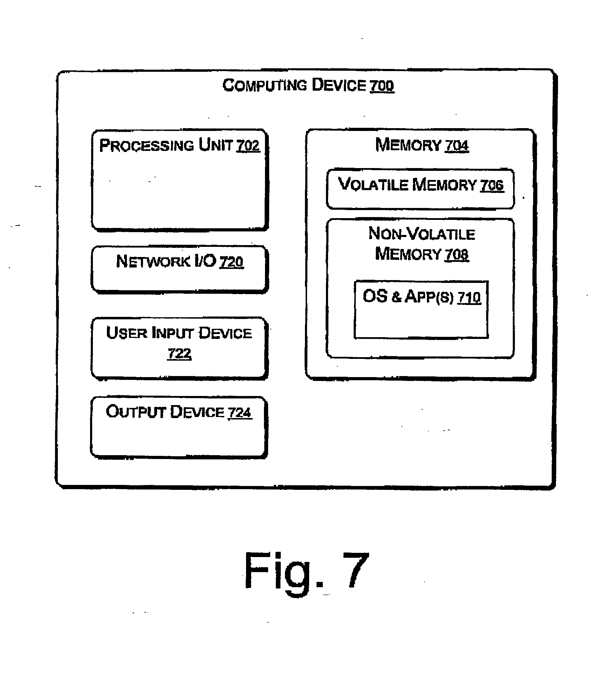

Fig. 7 shows an exemplary computing device 700 employed in the computing systems of the cardiac therapy network architecture 100. It includes a processing unit 702 and memory 704. Memory 704 includes both volatile memory 706 (e.g., RAM) and non-volatile memory 708 (e.g., ROM, EEPROM, Flash, disk, optical discs, persistent storage, etc.). An operating and system and various application programs 710 are stored in non-volatile memory 708. When a program is running, various instructions are loaded into volatile memory 706 and executed by processing unit 702. Examples of possible applications that may be stored and executed on the computing device include the knowledge worker specific applications 206 shown in Fig. 2.

The computing device 700 may further be equipped with a network I/O connection 720 to facilitate communication with a network. The network I/O 720 may be a wire-based connection (e.g., network card, modem, etc.) or a wireless connection (e.g., RF transceiver, Bluetooth device, etc.). The computing device 700 may also include a user input device 722 (e.g., keyboard, mouse, stylus, touch pad, touch screen, voice recognition system, etc.) and an output device 724 (e.g., monitor, LCD, speaker, printer, etc.).

Various aspects of the methods and systems described throughout this disclosure may be implemented in computer software or firmware as computer-executable instructions. When executed, these instructions direct the computing device (alone, or in concert with other computing devices of the system) to perform various functions and tasks that enable the cardiac therapy network architecture 100. Historical Cardiac-Status-and-Treatment Information

The data collected by one or more embodiments includes historical cardiac-status-and-treatment information for a specific patient. It may also include such information about multiple patients of a patent population. Historical cardiac-status-and-treatment information expressly includes information about patients (e.g., demographic data); cardiac status; cardiac treatment; cardiac equipment; etc.

The following are examples of data parameters that may be part of the historical cardiac-status-and-treatment information. These parameters may be used to correlate patient population data with a specific patient. This list is not exhaustive. Of course, other parameters may be used. Examples of patient-specific parameters:
- cardiac monitoring data and statistics;
- cardiac treatment data and statistics;
- ICTD model and serial number;
- ICTD battery status;
- waveform of treatment;
- shocking protocol;
- patient age;
- patient gender;
- patient activity patterns;
- patient weight;
- patient ethnic/nationality;
- patient genetics;
- patient temporal cycles;
- patient sleep/wake patterns; and
- patient medications.

### Operation

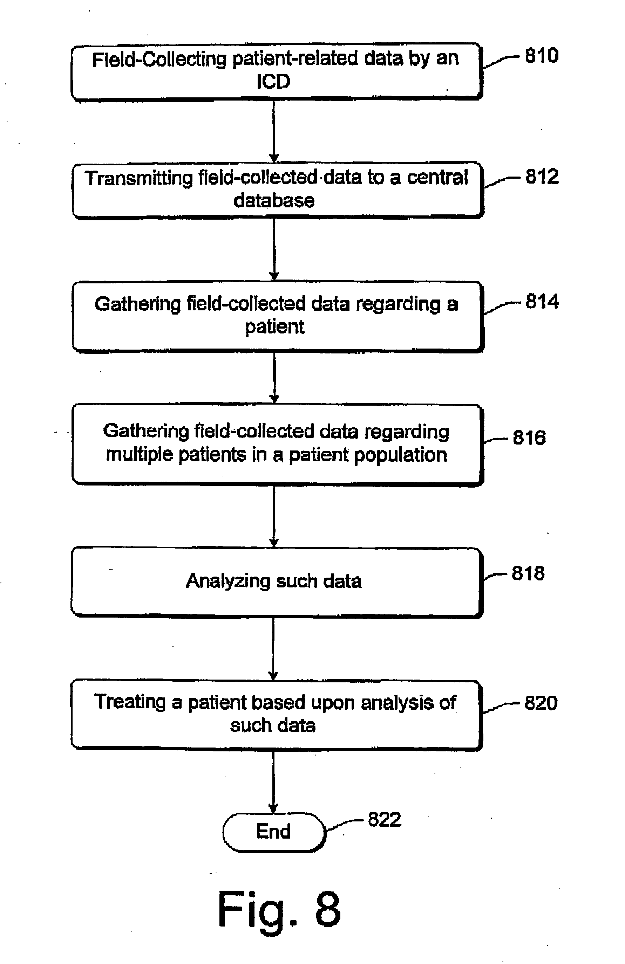

Fig. 8 shows methodological implementation of the exemplary feedback loop technology performed by the cardiac therapy network architecture 100 (or some portion thereof) and/or the patient feedback architecture 400 (or some portion thereof). This methodological implementation may be performed in software, hardware, or a combination thereof.

At 810 of Fig. 8, data is collected by a patient's ICTD. It is field-collected. This means that the data collected is primarily outside of the laboratory setting. Primarily, it is outside the controlled environments of the hospital or doctor's office. Although some of the data may have been collected during a programming session, the bulk is collected while the patient performs her normal daily activities.

At 812, this field-collected data is transmitted in accordance with the cardiac therapy network architecture 100.

At 814 of Fig. 8, the patient feedback architecture gathers the field-collected data regarding a specific patient. At 816, it gathers data about multiple patients in a patient population.

At 818, the patient feedback architecture analyzes the data. During such analysis, the patient feedback architecture searches for patterns in the data; correlations amongst the data; and the like. It uses techniques generally called knowledge discovery in databases (KDD). In the vernacular of those of ordinary skill in the art, these techniques are collectively called "data mining."

For example, statistical calculation may be performed to determine the best course of ICTD treatment for a patient having a particular age, gender, heart rate, etc.

Clinical trials may be performed since field results can be tracked and analyzed. For example, a medical team may experiment across a patient population with different rescue waveforms to determine which is best. Currently, biphasic waveform is used to defibrillate a patient. There is a belief that the leading edge of the biphasic waveform is the cause of great pain. Conventional techniques do not allow for the gathering experimental waveform data to determine if other waveforms may be more effective and/or less painful. Other waveforms may be stepped-up or rounded. It may be triphasic.

At 820, the treatment of a patient may be changed based upon the analysis of the patient's own data, of the patient population data, or a combination of both. This may be done automatically or manually. A signal may be sent back to the ICTD to alter the patient's treatment. For example, it may change the energy level used to rescue a patient suffering cardiac fibrillation. Alternatively, analysis of patient population data may lead to altered design and/or default programming of ICTDs.

The process ends at 822.

## Claims

1. Apparatus for administering cardiac therapy to a plurality of patients comprising: a plurality of implantable cardiac therapy devices (102) configured to be implanted in corresponding patients (104), a central database (120) and a communications link (112,114) configured to facilitate communications between the central database (120) and the plurality of implantable cardiac therapy devices (102), whereby the database (120) is operative to receive patient population data relating to therapy administered to the patients by the plurality of implantable cardiac therapy devices (102), **characterised in that** said central database is further adapted to process the collection of said patient population data to discover patterns, relationships, or correlations between such data, and to modify therapy of one or more of the implantable cardiac therapy devices (102) in response to the patterns, relationships, or correlations between the said patient population data.

2. Apparatus as claimed in Claim 1, **characterised in that** the data comprises historical cardiac-status-and-treatment information.

3. Apparatus as claimed in Claim 1 or Claim 2, **characterised in that** the data comprises historical cardiac-status-and-treatment information of a plurality of patients.

4. Apparatus as claimed in any preceding Claim, **characterized in that** the means carrying out the processing comprises a knowledge discovery in database (KDD) system.

5. Apparatus as claimed in any preceding Claim, **characterised in that** the data comprises one or more parameters selected from a group of parameters consisting of:
• cardiac monitoring data and statistics;
• cardiac treatment data and statistics;
• ICTD model and serial number;
• ICTD battery status;
• waveform of treatment;
• shocking protocol;
• patient age;
• patient gender;
• patient activity patterns;
• patient weight;
• patient ethnic/nationality;
• patient genetics;
• patient temporal cycles;
• patient sleep/wake patterns;
• patient medications.

## Patentansprüche

1. Vorrichtung zum Verabreichen von Herztherapie an eine Vielzahl von Patienten, die aufweist:
eine Vielzahl implantierbarer Herztherapievorrichtungen (102), die konfiguriert sind, um in entsprechende Patienten (104) implantiert zu werden,
eine zentrale Datenbank (120) und eine Kommunikationsverbindung (112, 114), die konfiguriert ist, um Kommunikationen zwischen der zentralen Datenbank (120) und der Vielzahl implantierbarer Herztherapievorrichtungen (102) zu ermöglichen,
wobei die Datenbank (120) betreibbar ist, um Patientenpopulationsdaten zu empfangen, die sich auf die Therapie beziehen, die den Patienten durch die Vielzahl implantierbarer Herztherapievorrichtungen (102) verabreicht wird,
**dadurch gekennzeichnet, dass** die zentrale Datenbank des Weiteren ausgelegt ist, um die erfassten Patientenpopulationsdaten zu verarbeiten, um Muster, Beziehungen oder Korrelationen zwischen diesen Daten zu erkennen, und um die Therapie einer oder mehrerer der implantierbaren Herztherapievorrichtungen (102) im Ansprechen auf die Muster, Beziehungen oder Korrelationen zwischen den Patientenpopulationsdaten zu modifizieren.

2. Vorrichtung nach Anspruch 1, **dadurch gekennzeichnet, dass** die Daten historische Herzstatus- und -behandlungsinformationen aufweisen.

3. Vorrichtung nach Anspruch 1 oder Anspruch 2, **dadurch gekennzeichnet, dass** die Daten historische Herzstatus- und -behandlungsinformationen einer Vielzahl von Patienten aufweisen.

4. Vorrichtung nach einem der vorhergehenden Ansprüche, **dadurch gekennzeichnet, dass** die Einrichtung, die die Verarbeitung durchführt, ein Knowledge Discovery in Database (KDD) -System aufweist.

5. Vorrichtung nach einem der vorhergehenden Ansprüche, **dadurch gekennzeichnet, dass** die Daten einen oder mehrere Parameter aufweisen, die aus einer Gruppe von Parametern ausgewählt sind, die besteht aus:
• Herzüberwachungsdaten und -statistiken;
• Herzbehandlungsdaten und -statistiken;
• ICTD Modell und Fabrikationsnummer;
• ICTD Batteriestatus;
• Verlauf der Behandlung;
• Schockprotokoll;
• Alter des Patienten;
• Geschlecht des Patienten;
• Aktivitätsmuster des Patienten;
• Gewicht des Patienten;
• Zugehörigkeit/Nationalität des Patienten;
• Erbanlagen des Patienten;
• Zeitzyklen des Patienten;
• Schlaf-/Wachmuster des Patienten;
• Medikamente des Patienten.

## Revendications

1. Appareil pour administrer un traitement cardiaque à une pluralité de patients comprenant : une pluralité de dispositifs de traitement cardiaque implantables (102), configurés pour être implantés dans des patients correspondants (104), une base de données centrale (120) et un lien de communication (112, 114) configuré pour faciliter les communications entre la base de données centrale (120) et la pluralité de dispositifs de traitement cardiaque implantable (102), moyennant quoi la base de données (120) est opérationnelle pour recevoir des données concernant une population de patients, relativement à un traitement administré auxdits patients par la pluralité de dispositifs de traitement cardiaque implantables (102), **caractérisé en ce que** ladite base de données centrale est en outre apte à traiter l'ensemble desdites données concernant une population de patients, afin de découvrir des tendances, des relations, ou des corrélations entre ces données, et de modifier le traitement d'un ou plusieurs des dispositifs de traitement cardiaque implantables (102) en réponse aux tendances, relations ou corrélations entre lesdites données concernant des populations de patients.

2. Appareil selon la revendication 1, **caractérisé en ce que** les données comprennent des informations historiques sur l'état cardiaque et le traitement.

3. Appareil selon la revendication 1 ou la revendication 2, **caractérisé en ce que** les données comprennent des informations historiques sur l'état cardiaque et le traitement d'une pluralité de patients.

4. Appareil selon l'une quelconque des revendications précédentes, **caractérisé en ce que** les moyens qui s'occupent du traitement comprennent une découverte de connaissances dans le système de base de données (KDD).

5. Appareil selon l'une quelconque des revendications précédentes, **caractérisé en ce que** les données comprennent un ou plusieurs paramètres choisis dans un groupe de paramètres constitués de :
- données et statistiques de monitoring cardiaque ;
- données et statistiques de traitement cardiaque ;
- modèle ICTD et numéro de série ;
- état de la batterie ICTD ;
- forme d'onde du traitement ;
- protocole de choc ;
- âge du patient ;
- sexe du patient ;
- types d'activité du patient ;
- poids du patient ;
- ethnie/nationalité du patient ;
- génétique du patient ;
- cycles temporels du patient ;
- cycles de sommeil/réveil du patient ;
- médicaments du patient.
